# EUROPEAN PATENT APPLICATION

(11) **EP 4 241 823 A1**
(43) Date of publication of application: **13.09.2023**
(21) Application number: 21889520.9
(22) Date of filing: 02.11.2021
(51) Int. Cl.: A61M 37/00, A61K 9/00

(54) **MICROSYSTEM FOR DELIVERING MULTIPLE MATERIALS**

(30) Priority: 04.11.2020 KR 20200146144; 05.08.2021 KR 20210102892
(71) Applicant: Cursus Bio Inc., Seoul 06170 (KR)
(72) Inventor: KIM, Yonghee, Seoul 04763 (KR); FAKHRAEI LAHIJI, Shayan, Seoul 04763 (KR)
(74) Representative: Frenkel, Matthias Alexander
(86) International application number: PCT/KR2021/015701
(87) International publication number: WO 2022/098055

(57) **Abstract**

A microsystem for delivering multiple materials is disclosed. The microsystem for delivering multiple materials can comprise: a microstructure which has microneedles formed on one surface of a base film and which is made of a first material; and a material accommodation part which is positioned in one region of the microstructure, and which has an accommodation space for accommodating a second material that differs from the first material.

## Description

### [Technical Field]

The present invention relates to a multi-material delivery microsystem, and more particularly, to a microsystem capable of delivering multiple materials by penetrating them into the skin.

### [Background Art]

Administration routes for delivering materials including pharmaceuticals or cosmetics to the body include oral, injectable, and transdermal types. Oral administration is the most common and convenient way and delivers active ingredients to the body in the form of capsules, tablets, or syrups. However, some of the active ingredients may be inactivated due to first-pass metabolism or the like in the liver. Therefore, to increase the effect of the active ingredients, they are administered by breaking through the skin, the body's defense barrier, in an injection form. The injection has the advantage of maintaining the activity of active ingredients delivered but has disadvantages such as the risk of infection, inaccurate dose administration, needle phobia, and pain.

Therefore, various microstructure-based transdermal active ingredient delivery systems, including minimally invasive biodegradable microneedles have been developed in order to overcome the limitations of existing oral and injectable administration routes. The biodegradable microstructure is a transdermal delivery system in which various active ingredients, including polymers, cosmetics, pharmaceuticals, etc., are formulated in the form of microneedles, inserted into the skin, and then dissolved by body fluids to deliver the loaded active ingredients painlessly.

However, biodegradable microstructures have limited loading (limited encapsulation capacity) of active ingredients. Since general biodegradable microstructures are manufactured in a microscopic size of tens to hundreds of microns, the loading amount is limited. Therefore, the amount of desired active ingredients cannot be delivered to the body, and there is a limitation in that the biodegradable microstructures are mainly applied to the cosmetic field.

In addition, biodegradable microstructures have inconvenience of attachment (inconvenience of continuous skin attachment). In general, biodegradable microstructures are attached to the skin in the form of patches and take several minutes to several hours to dissolve. Therefore, there are limitations such as the risk of causing various skin diseases including itching, dermatitis, and allergy due to the attachment of the patches and the inconvenience of attachment. In addition, regardless of the attachment time, there is a concern that the loaded active ingredients may not be efficiently delivered since the microstructures are not completely inserted into the skin due to the sharp tip and wide base of the microstructures.

In addition, biodegradable microstructures have a problem with inactivation when mixing multiple active ingredients (activity loss risk in multi-compound mixtures). Specifically, they may be inactivated due to various chemical reactions when mixed with biomaterials, polymers, and other active ingredients, depending on the nature of the active ingredients. Therefore, when multiple materials are loaded on the microstructures, there is a disadvantage in that the activation and stability of the active ingredients may be reduced.

In addition, biodegradable microstructures have limitations in loading hydrophobic active ingredients (limitations in encapsulation of poorly soluble compounds). The biodegradable microstructures are fabricated based on hydrophilic polymers. Therefore, active ingredients having hydrophobic molecules cannot be loaded on the microstructures, or a complicated targeting process is required to load the active ingredients.

### [Disclosure]

### [Technical Problem]

The present invention provides a multi-material delivery microsystem capable of maximizing the loading capacity and delivery volume of compounds to be delivered and maintaining the activity of the material.

In addition, the present invention provides a multi-material delivery microsystem capable of rapidly penetrating a material loaded on microneedles into the skin.

### [Technical Solution]

A multi-material delivery microsystem according to the present invention may include a microstructure in which microneedles are formed on one surface of a base film, and which is made of a first material; and a material accommodation part which is located in one region of the microstructure and has an accommodation space accommodating a second material different from the first material.

In addition, the base film may have one surface including: a central area; and a peripheral area which surrounds the central area and in which the microneedles are formed, and the material accommodation part may be located in the central area of the base film.

In addition, the accommodation space may be located so that the center of the bottom surface of the accommodation space is lower than one surface of the base film.

In addition, the material accommodation part may further include a ring-shaped partition wall which surrounds the accommodation space and is loacated so that an upper end thereof is lower than tips of the microneedles.

In addition, the partition wall may be provided as a corrugated wall having an upper end located at a first height in an expanded state and a second height lower than the first height in a contracted state.

In addition, the partition wall includes: a ring-shaped first partition wall layer; and a second partition wall layer which is located under the first partition wall layer and has a space formed therein capable of accommodating the first partition wall layer, wherein the first partition wall layer and the second partition wall layer may be switchable between a first state in which they are stacked in multiple stages and a second state in which the first partition wall layer is located inside the second partition wall layer.

In addition, the partition wall may include: support parts sequentially positioned to be spaced apart from each other along the circumference of the accommodation space; and connection parts which is positioned between the support parts and has a thickness thinner than those of the support parts.

In addition, the partition wall may gradually decrease in diameter and thickness as it goes from the lower end to the upper end.

In addition, the partition wall may gradually increase in diameter and gradually decrease in thickness as it goes from the lower end to the upper end.

In addition, the material accommodation part may further include a support plate which is located on the bottom of the base film and has the partition wall integrally formed in a central region thereof.

In addition, the support plate may have an inner part in which a storage space for storing the second material is formed, and an opening connecting the storage space and the accommodation space is formed.

In addition, the support plate has a storage space connected to the accommodation space and formed therein, the bottom surface of the storage space is provided as a film with a thin thickness, and the material accommodation part may further include a pushing part which punches the bottom surface of the storage space and is inserted into the storage space to push the second material stored in the storage space.

In addition, the support plate has a storage space connected to the accommodation space and formed therein, the bottom surface of the storage space is provided as a film which is convex downward and has a thin thickness, and when the bottom surface of the storage space is pressed from the outside, the bottom surface of the storage space may be pressed toward the accommodation space.

In addition, the multi-material delivery microsystem may further include: a membrane in which a plurality of storage spaces partitioned by partition walls are formed in the material accommodation part, and which is positioned in the opening of the base film; an outflow prevention film blocking an opening formed in the base film between the storage spaces and the membrane; and a punching rod of which a plurality of ends are each located in the storage spaces, respectively, and which can hit the outflow prevention film, wherein different materials are stored in the storage spaces, respectively, and the respective materials may be introduced into the membrane through holes formed in the outflow prevention film by hitting of the punching rod and mixed with each other.

### [Advantageous Effects]

According to the present invention, the material loaded on the microneedles and the material accommodated in the material accommodation part can be simultaneously penetrated into the skin.

In addition, according to the present invention, since the second material can quickly dissolve the first material, the first material can be penetrated into the skin in a short time.

### [Description of Drawings]

FIG. 1 is an exploded perspective view showing a multi-material delivery microsystem according to one embodiment of the present invention.
FIG. 2 is a cross-sectional view showing the microstructure of FIG. 1.
FIG. 3 is a view showing sample photographs of a microstructure fabricated according to an embodiment of the present invention.
FIG. 4 is a sample photograph showing a state in which a second material is loaded on the material accommodation part of the microstructure.
FIGS. 5 and 6 are views sequentially showing a process of delivering a first material and a second material into the skin using a multi-material delivery microsystem according to an embodiment of the present invention.
FIG. 7 is a cross-sectional view showing a multi-material delivery microsystem according to other embodiment of the present invention.
FIGS. 8 and 9 are cross-sectional views showing a multi-material delivery microsystem according to other embodiment of the present invention.
FIG. 10 is a perspective view showing a multi-material delivery microsystem according to another embodiment of the present invention.
FIG. 11 is an exploded perspective view showing the multi-material delivery microsystem of FIG. 10.
FIG. 12 is a cross-sectional view showing the multi-material delivery microsystem of FIG. 10.
FIG. 13 is a view showing a process of inserting microneedles into the skin using the multi-material delivery microsystem according to the embodiment of FIG. 12.
FIG. 14 is a cross-sectional view showing a partition wall structure according to other embodiment of the present invention.
FIG. 15 is a view showing an appearance in which the partition wall structure of FIG. 14 is deformed.
FIG. 16 is a cross-sectional view showing a partition wall structure according to other embodiment of the present invention.
FIG. 17 is a view showing an appearance in which the partition wall structure of FIG. 16 is deformed.
FIG. 18 is a cross-sectional view showing a partition wall structure according to other embodiment of the present invention.
FIG. 19 is a view showing an appearance in which the partition wall structure of FIG. 18 is deformed.
FIG. 20 is a cross-sectional view showing a partition wall structure according to other embodiment of the present invention.
FIG. 21 is a view showing an appearance in which the partition wall structure of FIG. 20 is deformed.
FIG. 22 is a view displayed on an XY plane showing a partition wall structure according to other embodiment of the present invention.
FIG. 23 is a view showing an appearance in which the partition wall structure of FIG. 22 is deformed.
FIG. 24 is a cross-sectional view showing a multi-material delivery microsystem according to another embodiment of the present invention.
FIG. 25 is a view showing a state of use of the multi-material delivery microsystem of FIG. 24.
FIG. 26 is a cross-sectional view showing a multi-material delivery microsystem according to another embodiment of the present invention.
FIG. 27 is a cross-sectional view showing a multi-material delivery microsystem according to another embodiment of the present invention.
FIG. 28 is a cross-sectional view showing a multi-material delivery microsystem according to another embodiment of the present invention.
FIG. 29 is a view showing an operation process of the multi-material delivery microsystem of FIG. 28.
FIGS. 30 to 32 are plan views showing microstructures according to various embodiments.

### [Best Mode for Carrying Out the Invention]

A multi-material delivery microsystem according to the present invention may include: a microstructure in which microneedles are formed on one surface of a base film, and which is made of a first material; and a material accommodation part which is located in one region of the microstructure and has an accommodation space accommodating a second material different from the first material.

### [Mode for Carrying Out the Invention]

Hereinafter, preferred embodiments of the present invention will be described in detail with reference to the accompanying drawings. However, the technical idea of the present invention is not limited to the embodiments described herein and may be embodied in other forms. Rather, the embodiments introduced herein are provided so that the disclosed content will be thorough and complete, and the spirit of the present invention will be sufficiently conveyed to those skilled in the art.

In the present specification, when an element is referred to as being on another element, it means that it may be directly formed on the other element or a third element may be interposed therebetween. Also, in the drawings, the thicknesses of films and regions are exaggerated for effective explanation of technical content.

In addition, although terms such as first, second, and third have been used in order to describe various elements in various embodiments of the present specification, these elements should not be limited by these terms. These terms have only been used in order to distinguish one element from another. Therefore, what is referred to as a first element in one embodiment may be referred to as a second element in another embodiment. Each embodiment described and exemplified herein also includes its complementary embodiments. In addition, in the present specification, 'and/or' has been used to mean including at least one of the elements listed before and after.

In the specification, expressions in the singular number include plural expressions unless the context clearly dictates otherwise. In addition, the terms "comprise" or "having" are intended to designate that a feature, number, step, component, or a combination thereof described in the specification exists, but should not be understood as excluding the possibility of the presence or addition of one or more other features, numbers, steps, components, or combinations thereof. In addition, in the present specification, "connection" is used as a meaning including both indirectly and directly connecting a plurality of components.

In addition, in the following description of the present invention, if it is determined that a detailed description of a related known function or configuration may unnecessarily obscure the subject matter of the present invention, the detailed description will be omitted.

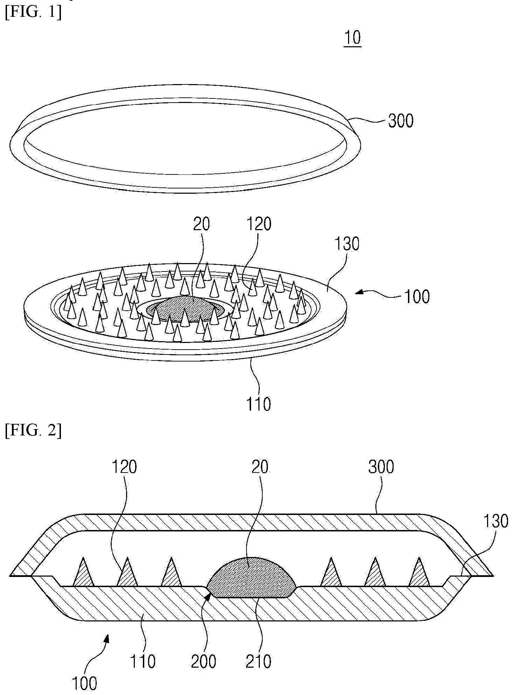

FIG. 1 is an exploded perspective view showing a multi-material delivery microsystem according to one embodiment of the present invention, and FIG. 2 is a cross-sectional view showing the microstructure of FIG. 1.

Referring to FIGS. 1 and 2 , the multi-material delivery microsystem 10 may deliver a first material and a second material to the skin of a user.

The first material is a biodegradable material that is dissolved after being penetrated into the skin, and may be a material that is non-toxic to the human body, chemically inert, and non-immunogenic. The first material may be decomposed by body fluids, microorganisms, or the like in vivo.

According to one embodiment, the first material may include hyaluronic acid, polyester, polyhydroxyalkanoate (PHAs), poly(a-hydroxy acid), poly(β-hydroxy acid), poly(3-hydroxybutyrate-co-hydroxyvalerate (PHBV), poly(3-hydroxypropionate) (PHP), poly(3-hydroxyhexanoate) (PHH), poly(4-hydroxyacid), poly(4-hydroxybutyrate), poly(4-hydroxyvalerate), poly(4-hydroxyhexanoate), poly(ester amide)s, polycaprolactone, polylactide, polyglycolide, poly(lactide-co-glycolide) (PLGA), polydioxanone, polyorthoesters, polyetheresters, polyanhydrides, poly(glycolide-co-trimethylene carbonate), polyphosphoesters, polyphosphoester urethanes, poly(amino acid), polycyanoacrylates, poly(trimethylene carbonate), poly(imino carbonates), poly(tyrosine carbonate), polycarbonate, poly(tyrosine arylate), poly(alkylene oxalate), polyphosphagens, phytohemagglutinin-polyethylene glycol (PHA-PEG), ethylene vinyl alcohol copolymer (EVOH), polyurethane, silicone, polyester, polyolefin, polyisobutylene and ethylene-alpha olefin copolymer, styrene-isobutylene-styrene triblock copolymer, acrylic polymers and copolymers, vinyl halide polymers and copolymers, polyvinyl chloride, polyvinyl ether, polyvinyl methyl ether, polyvinylidene halide, polyvinylidene fluoride, polyvinylidene chloride, polyfluoroalkanes, polyperfluoroalkanes, polyacrylonitriles, polyvinyl ketones, polyvinylaromatics, polystyrenes, polyvinyl esters, polyvinyl acetates, ethylene-methyl methacrylate copolymers, acrylonitrile-styrene copolymers, ABS resin and ethylenevinyl acetate copolymer, polyamide, alkyd resin, polyoxymethylene, polyimide, polyether, polyacrylate, polymethacrylate, poly(acrylic acid-co-maleic acid), chitosan, dextran, cellulose, heparin, alginate, inulin, starch, or glycogen, or may include one or more selected from the group consisting of hyaluronic acid, polyester, polyhydroxyalkanoate (PHAs), poly(o-hydroxy acid), poly(β-hydroxy acid), poly(3-hydroxybutyrate-co-hydroxyvalerate (PHBV), poly(3-hydroxypropionate) (PHP), poly(3-hydroxyhexanoate) (PHH), poly(4-hydroxyacid), poly(4-hydroxybutyrate), poly(4-hydroxyvalerate), poly(4-hydroxyhexanoate), poly(ester amide)s, polycaprolactone, polylactide, polyglycolide, poly(lactide-co-glycolide) (PLGA), polydioxanone, polyorthoesters, polyetheresters, polyanhydrides, poly(glycolide-cotrimethylene carbonate), polyphosphoesters, polyphosphoester urethanes, poly(amino acid), polycyanoacrylates, poly(trimethylene carbonate), poly(imino carbonates), poly(tyrosine carbonate), polycarbonate, poly(tyrosine arylate), poly(alkylene oxalate), polyphosphagens, phytohemagglutinin-polyethylene glycol (PHA-PEG), chitosan, dextran, cellulose, heparin, alginate, inulin, starch, and glycogen.

According to another embodiment, the first material may be a drug. A drug means a concept in a broad sense, and may include not only therapeutic agents for treatment purpose in a narrow sense, but also energy, nanocomponents, cosmetic ingredients (e.g., anti-wrinkle agents, skin aging inhibitors, and skin whitening agents), cell culture solutions, and the like. Specifically, the therapeutic agents may include chemical drugs, protein/peptide drugs, peptide drugs, nucleic acid molecules for gene therapy, and the like.

For example, the therapeutic agents may include anti-inflammatory drugs, analgesics, anti-arthritic drugs, antispasmodics, anti-depressants, antipsychotic drugs, tranquilizers, anti-anxiety drugs, narcotic antagonists, anti-parkinsonian drugs, cholinergic agonists, anti-cancer drugs, anti-angiogenic inhibitors, and immunosuppressive drugs. , antiviral drugs, antibiotics, appetite suppressants, analgesics, anticholinergics, antihistamines, antimigraine drugs, hormones, coronary vascular, cerebrovascular or peripheral vascular vasodilators, contraceptives, antithrombotic drugs, diuretics, antihypertensives, cardiovascular disease treatment, etc.

In particular, protein/peptide drugs may include hormones, hormone analogues, enzymes, enzyme inhibitors, signal transduction proteins or parts thereof, antibodies or parts thereof, single-chain antibodies, binding proteins or binding domains thereof, antigens, attachment proteins, structural proteins, regulatory proteins, toxin proteins, cytokines, transcriptional regulatory factors, blood coagulation factors, and vaccines. More specifically, the protein/peptide drugs may include insulin, insulin-like growth factor 1 (IGF-1), growth hormone, erythropoietin, granulocyte-colony stimulating factors (G-CSFs), granulocyte/macrophagecolony stimulating factors (GM-CSFs), interferon alpha, interferon beta, interferon gamma, interleukin-1 alpha and beta, interleukin-3, interleukin-4, interleukin-6, interleukin-2, epidermal growth factors (EGFs), calcitonin, adrenocorticotropic hormone (ACTH), tumor necrosis factor (TNF), atobisban, buserelin, cetrorelix, deslorelin, desmopressin, dynorphin A (1-13), elcatonin, eleidosin, eptifibatide, growth hormone releasing hormone-II (GHRH-II), gonadorelin, goserelin, histrelin, leuprorelin, lypressin, octreotide, oxytocin, pitressin, secretin, sincalide, terlipressin, thymopentin, thymosin α1, triptorelin, bivalirudin, carbetocin, cyclosporine, exedine, lanreotide, luteinizing hormone-releasing hormone (LHRH), nafarelin, parathyroid hormone, pramlintide, enfuvirtide (T-20), thymalfasin, and ziconotide.

In addition, energy may include thermal energy, light energy, electrical energy, and the like. For example, microstructures in photodynamic therapy may be used to induce light to a specific part in the body so that light can directly act on tissues or act on mediators such as lightsensitive molecules.

In addition, the first material may be a water-soluble drug. The first material may be in a solid, liquid, powder or highly concentrated state.

The second material may be a drug like the first material, a material capable of dissolving the first material, or a material capable of increasing the delivery efficiency and speed of the first material.

As the second material is delivered to the body together with the first material, various efficacies may be expected. In addition, the interaction between the first material and the second material controls the dissolution rate of the first material in the body so that the efficacy can be maximized. In addition, since the second material has a certain strength of adhesion due to its characteristics, the multi-material delivery microsystem may be closely contacted with the skin.

According to one embodiment, the second material may include a fat-soluble material. Specifically, the second material may include one or more fat-soluble materials selected from the group consisting of horse oil, vitamin A and its derivatives, vitamin D and its derivatives, vitamin E and its derivatives, vitamin K and its derivatives, organic sunscreens, and fat-soluble plant extracts.

According to another embodiment, the second material may be a material capable of dissolving the first material or rapidly adjusting the dissolution rate. Specifically, the second material may include one or more selected from the group consisting of water, C₁-C₄ anhydrous or hydrous lower alcohols, benzene, toluene, xylene, hexane, chloroform, ether, acetone, and amine.

According to another embodiment, the second material may include a material for protecting the skin. Specifically, the second material may include a moisturizer, a skin cleanser, a disinfectant, a skin restoration agent, a soothing agent, etc. The moisturizer may include glycerin, propylene glycol, sorbitol, amino acids, butylene glycol, hyaluronic acid, etc., the skin cleanser may include various polymer compounds, polyols, ester oils, oils and fats, silicone compounds, etc., the disinfectant may include C₁-C₄ lower alcohols, salicylic acid, camphor, etc., and the skin restoration agent may include ceramide, almond, organ vegetable oil, shea butter, etc.

The multi-material delivery microsystem 10 includes a microstructure 100, a material accommodation part 200, and a protective cap 300 .

The microstructure 100 can be made of a first material, and microneedles 120 are formed on one surface of a base film 110.

The base film 110 is provided to a predetermined width in the form of a film with a thin thickness. The base film 110 may be provided in a circular or polygonal shape. The base film 110 may have a width of 1 to 200 mm² or 1 to 100 mm².

The microneedles 120 protrude from one surface of the base film 110 to a predetermined height, and their ends are provided in a sharp form to facilitate penetration into the skin. According to the embodiment, the microneedles 120 may have a height of 10 to 20,000 µm, 10 to 10,000 µm, 20 to 10,000 µm, 30 to 8,000 µm, or 50 to 2,000 µm. Each region of the tips of the microneedles 120 may be provided to have a minimum diameter of 1 to 500 µm, 2 to 300 µm, or 5 to 100 µm, and a maximum diameter of 50 to 1,000 µm.

The microneedles 120 may be provided at preset intervals on the remnant area of one surface of the base film 110 except for the area where the material accommodation part 200 is located. According to the embodiment, the microneedles 120 may be provided to surround the central area of the base film 110. The microneedles 120 may be formed to a density of 1 to 5 needles/mm².

A skin isolation wall 130 may be formed in the microstructure 100 along an edge region of the base film 110. The skin isolation wall 130 has an upper end higher than one surface of the base film 110 and a height lower than the ends of the microneedles 120.

The material accommodation part 200 provides an accommodation space for accommodating the second material 20. The material accommodation part 200 may be formed in the central region of the microstructure 100. According to the embodiment, the material accommodation part 200 may be integrally formed with the base film 110 in the central area of the base film 110. The material accommodation unit 200 may have a height of a bottom surface of the accommodation space 210 thereof lower than one surface of the base film 110 on which the microneedles 120 are formed. The bottom surface of the accommodation space 210 may have a downwardly concave shape.

The protective cap 300 has a width corresponding to or greater than that of the microstructure 100 and covers the top of the microstructure 300 to block the microneedles 120 from being exposed to the outside. The protective cap 300 is combined with the microstructure 100 when the multi-material delivery microsystem 10 is stored, and is removed from the microstructure 100 during medical procedure.

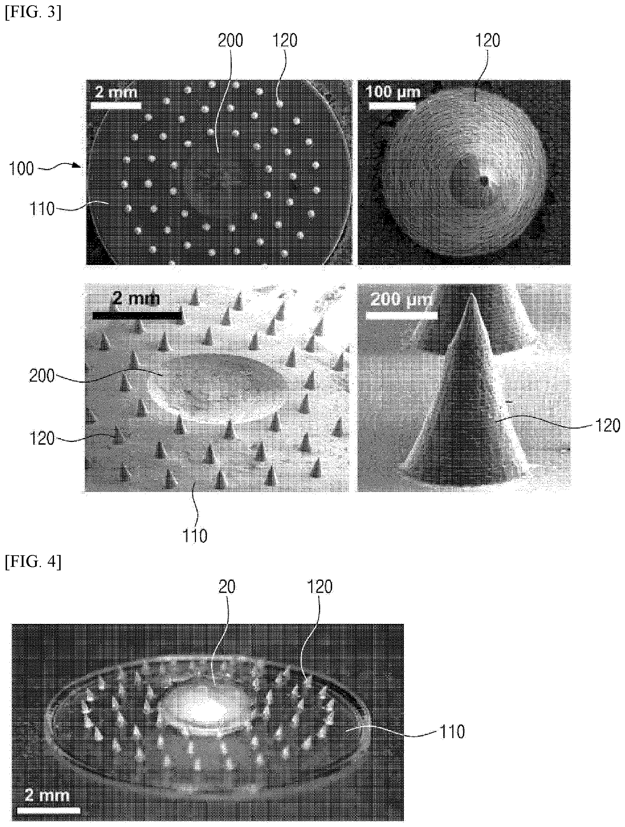

FIG. 3 is a view showing sample photographs of a microstructure fabricated according to an embodiment of the present invention, and FIG. 4 is a sample photograph showing a state in which a second material is loaded on the material accommodation part of the microstructure.

Referring to FIGS. 3 and 4, it can be confirmed that the material accommodation part 200 is formed in a concave groove shape in the central region of the microstructure 100, and the second material 20 is loaded on the material accommodation part 200.

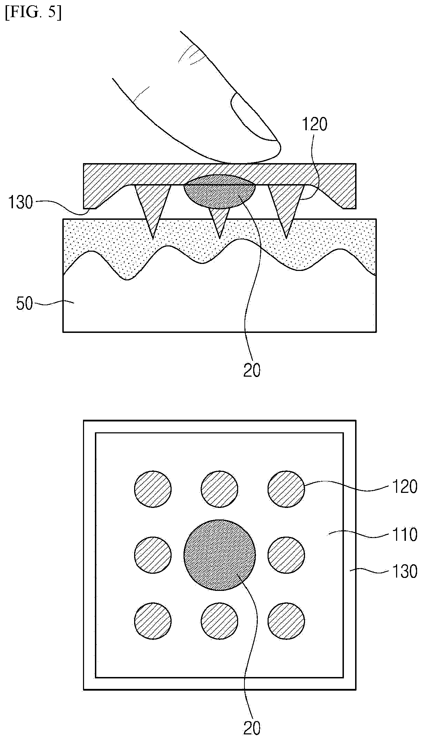

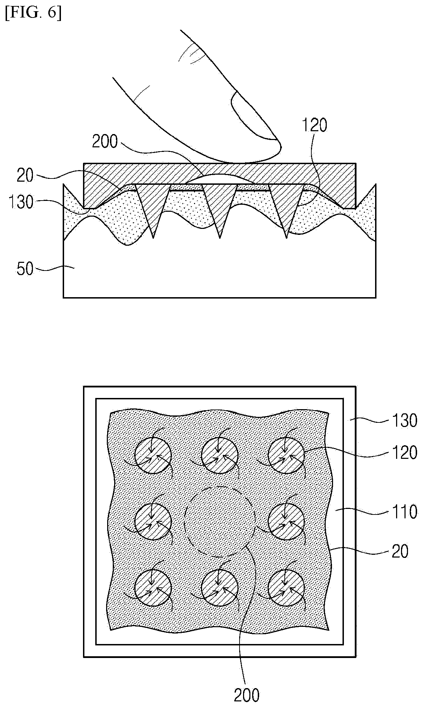

FIGS. 5 and 6 are views sequentially showing a process of delivering a first material and a second material into the skin using a multi-material delivery microsystem according to an embodiment of the present invention.

Referring to FIG. 5, after removing the protective cap 300, the user presses the rear surface of the base film 110 with a finger in a state in which the multi-material delivery microsystem 10 is positioned so that the microneedles 120 come into contact with the skin 50. In this process, the microneedles 120 penetrate the skin 40, and the second material 20 accommodated in the material accommodation part 200 spreads to the entire area of the base film 110 through the space between the base film 110 and the skin 40. The second material 20 is blocked from leaking out of the microstructure 100 due to the skin isolation wall 130 pressing the peripheral area of the skin.

As time goes by, the first material constituting the microneedles 120 penetrates into the skin while being dissolved. The second material 20 penetrates into the skin together with the first material and promotes dissolution of the first material. In addition, the second material 20 protects the skin surface penetrated by the microneedles 120 or suppresses the occurrence of irritation.

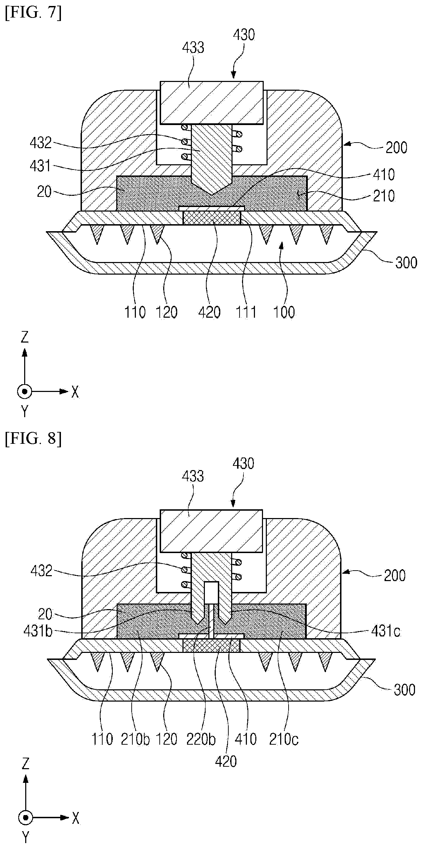

FIG. 7 is a cross-sectional view showing a multi-material delivery microsystem according to other embodiment of the present invention.

Referring to FIG. 7, a space 210 for storing the second material 20 is formed inside the material accommodation part 200. In addition, an opening 111 is formed in the central area of the base film 110.

The multi-material delivery microsystem 10 further includes an outflow prevention film 410, a membrane 420, a punching part 430, and an elastic spring 440.

The outflow prevention film 410 is a film with a thin thickness, and blocks the opening 111 of the base film 100. The outflow prevention film 410 is provided as a material that is not dissolved by the second material 20, and outflow of the second material 20 is blocked by the outflow prevention film 410. The outflow prevention film 410 may be provided as a synthetic resin or a rubber material.

The membrane 420 is located in the opening 111 of the base film 100 and is provided as a porous material. The membrane 420 controls the rate at which the second material 20 is flown out. The second material 20 may be slowly introduced toward the microneedles 120 by the membrane 420. According to the embodiment, the membrane 420 may be provided of a ceramic or fiber material.

The punching part 430 opens the outflow prevention film 410 by a user's manipulation. The punching part 430 includes a punching rod 431, an elastic spring 432, and an operation button 433.

The punching rod 431 has an end positioned in the inner space 210 of the material accommodation part 200 and has a sharp tip.

The elastic spring 432 is located outside the material accommodation part 200, and the punching rod 431 is inserted thereinto.

The operation button 433 is coupled with the upper end of the punching rod 431.

When the user presses the operation button 433, while the punching rod 431 moves forward, the sharp end strikes the outflow prevention film 410. As a result, a hole is formed in the outflow prevention film 410, and the second material 20 is introduced toward the membrane 420 through the hole. The second material 20 is absorbed by the membrane 420 and introduced toward the microneedles 120 as the outflow rate is controlled.

When the force pressing the operation button 433 is removed, the punching rod 431 is moved to its original position by the elastic force of the elastic spring 432.

The multi-material delivery microsystem 10 according to this embodiment may be suitable for use when the fluidity of the second material 20 is high.

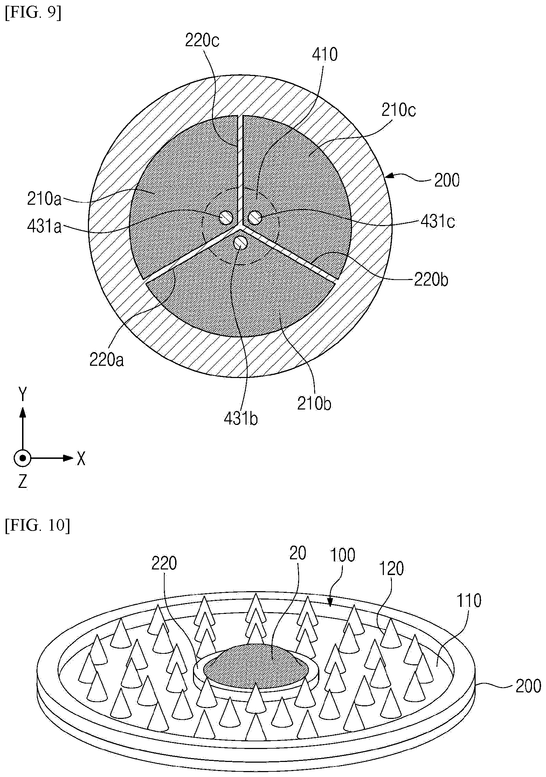

FIGS. 8 and 9 are cross-sectional views showing a multi-material delivery microsystem according to other embodiment of the present invention.

Referring to FIGS. 8 and 9, partition walls 220a to 220c are formed in the material accommodation part 200. The partition walls 220a to 220c divide the inner space of the material accommodation part 200 into a plurality of spaces 210a to 210c. Different materials among the aforementioned second materials are provided in each of the spaces 210a to 210c.

The punching rods 431a to 431c have their ends separated into a number corresponding to the number of the spaces 210a to 210c, and hit the outflow prevention film 410 in the respective spaces 210a to 210c.

With hitting of the outflow prevention film 41, materials stored in the respective spaces 210a to 210c flow out through the opening 111 and are absorbed into the membrane 420. After the respective materials are mixed in the membrane 420, they as the mixed materials are provided to the microneedles 120.

The multi-material delivery microsystem 10 according to the embodiment of the present invention divides and stores materials whose effects may be reduced or whose properties may change when they are stored as mixed materials in separate spaces 210a to 210c, and creates mixed materials when penetrating the materials into the skin.

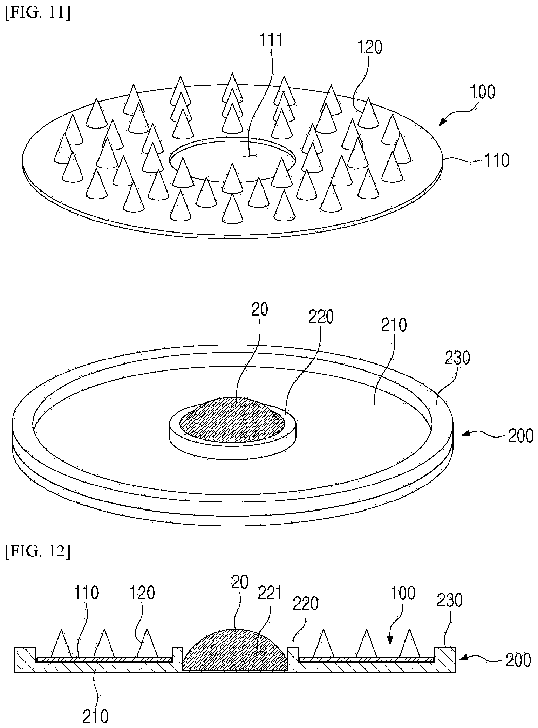

FIG. 10 is a perspective view showing a multi-material delivery microsystem according to another embodiment of the present invention, FIG. 11 is an exploded perspective view showing the multi-material delivery microsystem of FIG. 10, and FIG. 12 is a cross-sectional view showing the multi-material delivery microsystem of FIG. 10.

Referring to FIGS. 10 to 12, when the second material 20 is a material that dissolves the first material, if the second material 20 is stored in a state of contact with the microstructure 100, the microstructure 100 may be dissolved. In order to prevent this, it is necessary to separate the second material 20 from the microstructure 100.

The multi-material delivery microsystem 10 according to the embodiment of the present invention includes a microstructure 100 and a material accommodation part 200.

The microstructure 100 is made of the above-described first material and includes the base film 110 and the microneedles 120.

The base film 110 may have the thickness and width described in the embodiment of FIG. 1. One surface of the base film 110 includes a central area and a peripheral area.

An opening 111 is formed in the central area of the base film 110. The peripheral area is an area surrounding the central area, and the microneedles 120 are formed. The microneedles 120 have the same structures and sizes as those of the microneedles described in FIG. 1.

The material accommodation part 200 is provided separately from the microstructure 100 and is provided as a material that is not dissolved by the second material 20. According to the embodiment, the material accommodation part 200 may be made of a material that is flexible and human body-friendly. The material accommodation part 200 may be made of a synthetic resin, silicone, or rubber material.

The material accommodation part 200 includes a support plate 210, a partition wall 220, and a skin isolation wall 230.

The support plate 210 is provided as a thin-thickness plate having a width and a shape corresponding to those of the base film 110.

The partition wall 220 is formed in the central region of the support plate 210. The partition wall 220 is a ring shape having an outer diameter corresponding to the opening 111 of the base film 110, and is provided at a predetermined height from one surface of the support plate 210. The inner space of the partition wall 220 is provided as an accommodation space 221 capable of accommodating a second drug 20. A bottom surface of the accommodation space 221 may be provided as a concave curved surface. When the microstructure 100 is placed on one surface of the support plate 210, the partition wall 220 is inserted into the opening 111 of the base film 110. In addition, the upper end of the partition wall 220 is higher than one surface of the base film 110 and has a height lower than those of ends of the microneedles 120.

The skin isolation wall 230 is provided at a predetermined height along the edge region of the support plate 210. The skin isolation wall 230 has an upper end higher than one surface of the base film 110 and a height lower than those of the ends of the microneedles 120. The skin isolation wall 230 may have various widths in the radial direction of the support plate 110. In addition, one side surface of the skin isolation wall 230 facing the partition wall 220 may be provided as a curved surface.

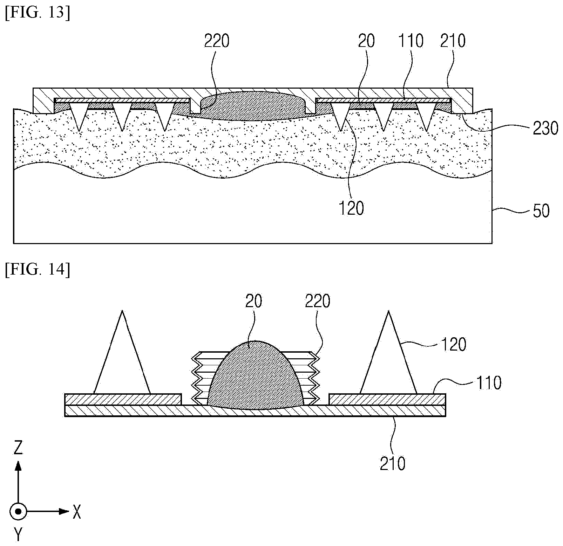

FIG. 13 is a view showing a process of inserting microneedles into the skin using the multi-material delivery microsystem according to the embodiment of FIG. 12.

First, referring to FIG. 12, the second material 20 is stored in a state in which contact with the microstructure 100 is blocked by the partition wall 220 of the material accommodation part 200. Therefore, during the storage period, the microstructure 100 may maintain its original appearance without being dissolved.

Referring to FIG. 13, when the user wants to insert the microneedles 120 into the skin 50, the microneedles 120 are penetrated into the skin by pressing the rear surface of the support plate 210 in a state in which the microstructure 100 is in contact with the skin 50. In this process, the second material 20 flows out through the gap between the skin 50 and the partition wall 220 and spreads toward the microneedles 120. In addition, leakage to the outside is blocked by the skin isolation wall 230 pressing the peripheral area. The second material 20 is in contact with the microneedles 120 to promote decomposition of the microneedles 120. Due to this, the first material may penetrate the skin 50 within a short time.

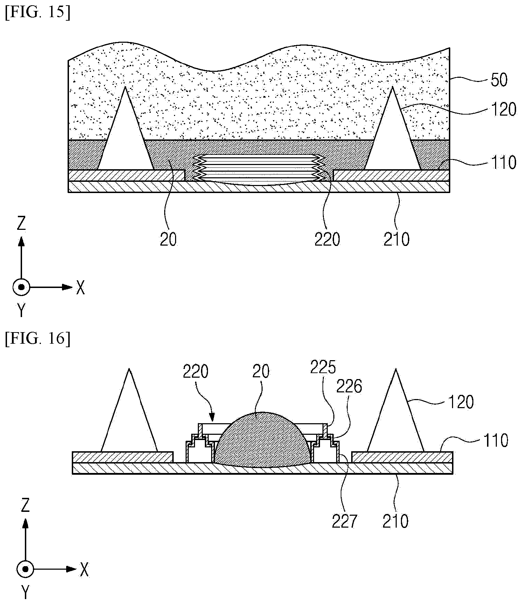

FIG. 14 is a cross-sectional view showing a partition wall structure according to other embodiment of the present invention, and FIG. 15 is a view showing an appearance in which the partition wall structure of FIG. 14 is deformed.

Referring to FIG. 14, the partition wall 220 may have a thin thickness and may have corrugations formed in a height direction. The partition wall 220 has its upper end located at a first height in a state where the corrugations are inflated, and accommodates the second material in the accommodation space 221 of the inner side.

Referring to FIG. 15, in the process of inserting the microneedles 120 into the skin 50, the partition wall 220 is pressed against the skin 50 and contracted. Due to this, the upper end of the partition wall 220 is located at a second height lower than the first height, and the second material may easily spread through the gap between the skin 50 and the partition wall 220.

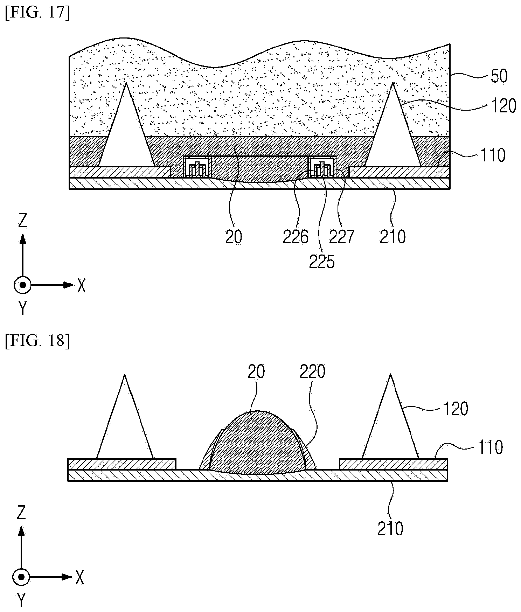

FIG. 16 is a cross-sectional view showing a partition wall structure according to other embodiment of the present invention, and FIG. 17 is a view showing an appearance in which the partition wall structure of FIG. 16 is deformed.

Referring to FIG. 16, the partition wall 220 has a structure in which a plurality of ring-shaped partition wall layers 225 to 227 are stacked in multiple stages. At least two partition wall layers 225 to 227 may be provided. In this embodiment, the provision of three partition wall layers 225 to 227 is described as an example.

The first partition wall layer 225 has a ring shape and is located in the upper end of the partition wall 220.

The second partition wall layer 226 is located below the first partition wall layer 225, and a space capable of accommodating the first partition wall layer 225 is formed therein.

The third partition wall layer 227 is located below the second partition wall layer 226, and a space capable of accommodating the second partition wall layer 226 is formed therein. The third partition wall layer 227 is coupled to one surface of the support plate 210.

The partition wall 220 can switch between the first state and the second state. The first state is a state in which the first to third partition wall layers 225 to 226 are sequentially stacked, and the upper end of the first partition wall layer 225 is positioned at the first height. In the second state, the first partition wall layer 225 is located in the inner space of the second partition wall layer 226, and the second partition wall layer 226 is located in the inner space of the third partition wall layer 227. Therefore, the upper end of the partition wall 220 has a second height lower than the first height, that is, a height that is the same as that of the upper end of the third partition wall layer 227.

The partition wall 220 accommodates the second material 20 therein in the first state. In addition, in the process of inserting the microneedles 120 into the skin 50, it is pressed to the skin 50 and converted to the second state. In the second state, the second material 20 may easily spread through the gap between the skin 50 and the partition wall 220.

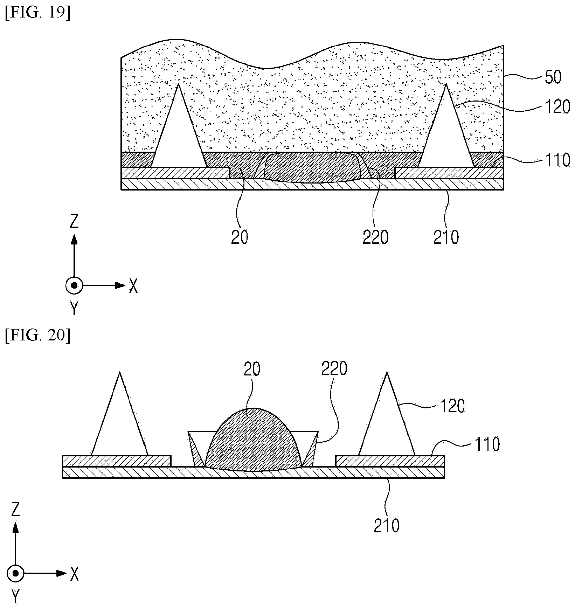

FIG. 18 is a cross-sectional view showing a partition wall structure according to other embodiment of the present invention, and FIG. 19 is a view showing an appearance in which the partition wall structure of FIG. 18 is deformed.

Referring to FIGS. 18 and 19, the diameter and thickness of the partition wall 220 gradually decrease as it goes from the lower end adjacent to the support plate 210 to the upper end. The lower end of the partition wall 220 has a first diameter, and the upper end has a second diameter smaller than the first diameter. In general, the partition wall 220 has a dome structure. The second material 20 is accommodated inside the partition wall 220.

In the process of inserting the microneedles 120 into the skin 50, the partition wall 220 is sequentially pressed against the skin 50 from the upper end. As a result, the partition wall 220 is bent toward the accommodation space 221. Since the upper end of the partition wall 220 is provided at a thin thickness, it may be easily pressed with a small force. In this process, the second material 20 may easily spread through the gap between the skin 50 and the partition wall 220.

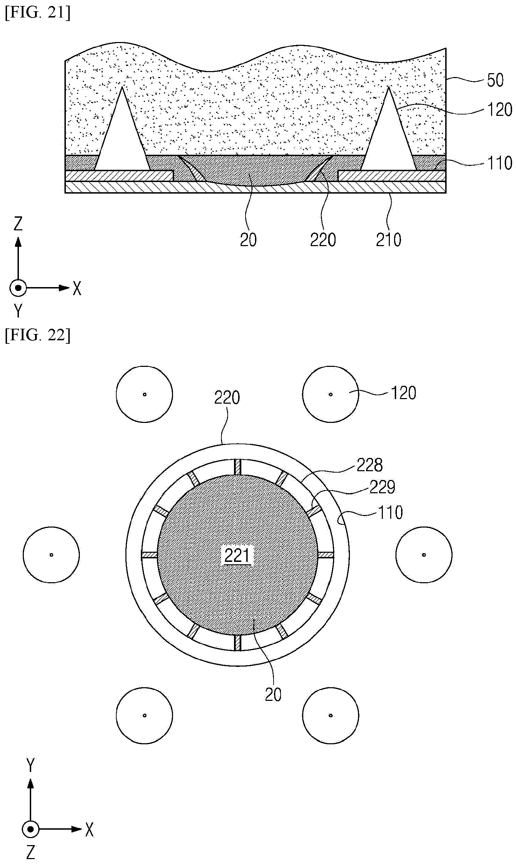

FIG. 20 is a cross-sectional view showing a partition wall structure according to other embodiment of the present invention, and FIG. 21 is a view showing an appearance in which the partition wall structure of FIG. 20 is deformed.

Referring to FIGS. 20 and 21, the diameter of the partition wall 220 gradually increases as it goes from the lower end adjacent to the support plate 210 to the upper end, and the thickness thereof gradually decreases. The lower end of the partition wall 220 has a first diameter, and the upper end thereof has a second diameter larger than the first diameter. The second material 20 is accommodated inside the partition wall 220.

In the process of inserting the microneedles 120 into the skin 50, the skin 50 is sequentially pressed from the upper end of the partition wall 220. The partition wall 220 is bent outward of the accommodating space 221 so that the diameter of the upper end thereof becomes larger. Since the upper end of the partition wall 220 is provided at a thin thickness, it may be easily pressed with a small force. In this process, the second material 20 may easily spread through the gap between the skin 50 and the partition wall 220.

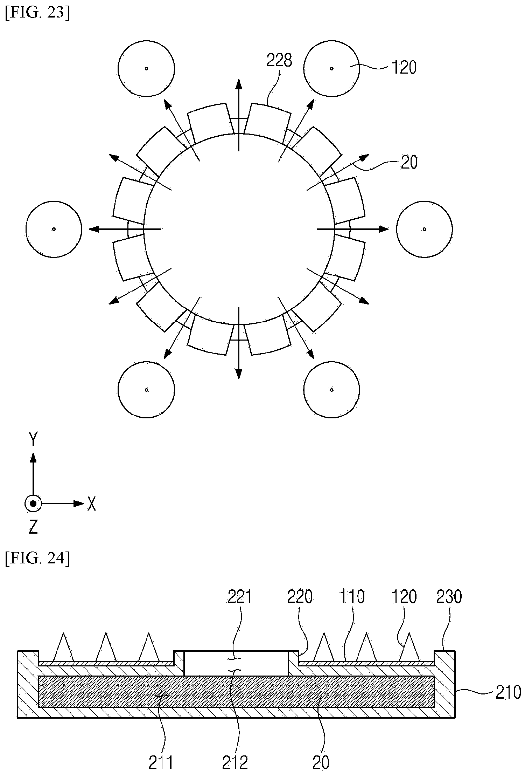

FIG. 22 is a view displayed on an XY plane showing a partition wall structure according to other embodiment of the present invention, and FIG. 23 is a view showing an appearance in which the partition wall structure of FIG. 22 is deformed.

Referring to FIGS. 22 and 23, the partition wall 220 is provided by combining support parts 228 and connection parts 229.

The support parts 228 are an arc-shaped plate having a predetermined length, and a plurality of them are sequentially disposed to be spaced apart from each other along the circumference of the accommodation space 221.

The connection parts 229 connect both side portions of the adjacent support parts 228 between the support parts 228 to each other. The connection parts 229 have thicknesses thinner than those of the support parts 228. The connection parts 229 have thicknesses of the extent sufficient to be easily torn when an external force is applied.

As described above, the partition wall 220 is provided as a combination structure of the support parts 228 and the connection parts 229 and accommodates the second material 20 in the accommodation space 221.

In the process of inserting the microneedles 120 into the skin 5, the upper ends of the support parts 228 are pressed by the skin 50 and are bent toward the outside of the accommodation space 221. In this process, the connection parts 229 are torn, and the second material 20 is introduced toward the microneedles 120 through the space between the support parts 228 in which the connection parts 229 are torn.

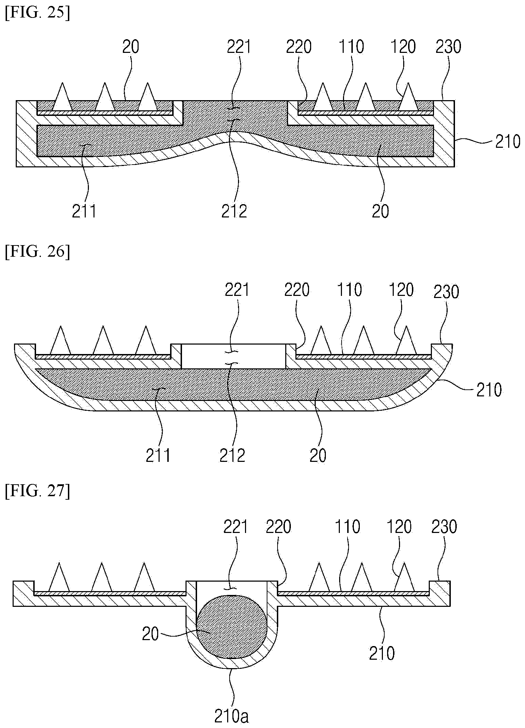

FIG. 24 is a cross-sectional view showing a multi-material delivery microsystem according to another embodiment of the present invention, and FIG. 25 is a view showing a state of use of the multi-material delivery microsystem of FIG. 24.

Referring to FIG. 24, a storage space 211 capable of storing the second material 20 is formed inside the support plate 210. The bottom surface of the storage space 211 may be provided flat. The storage amount of the second material 20 may be increased by forming the separate storage space 211 inside the support plate 210. In addition, an opening 212 is formed in a central region of the support plate 210 on which the partition wall 220 is formed. The opening 212 is connected to the storage space 211 and the accommodation space 221 inside the partition wall 220, and provides a passage through which the second material 20 stored in the storage space 211 may flow out.

Referring to FIG. 25, since the support plate 210 is provided as a flexible material, when the user presses the bottom surface of the support plate 210, the bottom surface is deformed toward the opening 211. This facilitates the outflow of the second material 20 stored in the storage space 211 through the opening 212.

FIG. 26 is a cross-sectional view showing a multi-material delivery microsystem according to another embodiment of the present invention.

Referring to FIG. 26, unlike the embodiment of FIG. 24, the bottom surface of the storage space 211 may be provided as a concave curved surface. The concave curved surface facilitates the outflow of the second material 20 toward the opening 212.

FIG. 27 is a cross-sectional view showing a multi-material delivery microsystem according to another embodiment of the present invention.

Referring to FIG. 27, the support plate 210 is provided as a curved surface in which the bottom surface 210a of the region where the partition wall 220 is located is convex downward. The bottom surface 210a may be provided in various depths depending on the storage capacity of the second material 20. The user may press the bottom surface 210a to supply the second material 20 toward the microneedles120.

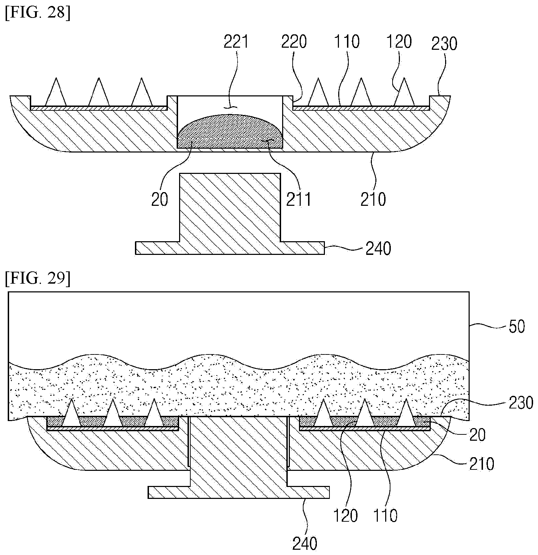

FIG. 28 is a cross-sectional view showing a multi-material delivery microsystem according to another embodiment of the present invention, and FIG. 29 is a view showing an operation process of the multi-material delivery microsystem of FIG. 28.

Referring to FIGS. 28 and 29, in the support plate 210, the bottom surface of the region in which the storage space is formed is provided as a film with a thin thickness.

The multi-material delivery microsystem 10 further includes a pushing part 240. The pushing part 240 can be inserted into the storage space 211. When a user pushes the front end of the pushing part 240 against the bottom surface of the storage space 211, the bottom surface provided as a thin film is torn and the pushing part 240 is inserted into the storage space 211. The second material 20 stored in the storage space 211 may be pushed by the pushing part 240 and supplied toward the microneedles 120.

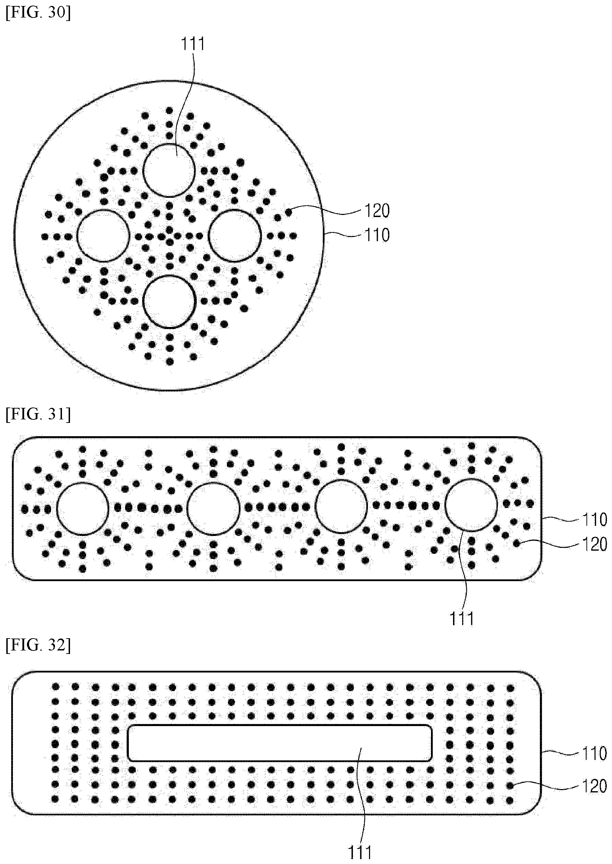

FIGS. 30 to 32 are plan views showing microstructures according to various embodiments.

First, referring to FIG. 30, it has been described in the above-described embodiment that the opening 111 is formed in the central area of the base film 110, but a plurality of openings 111 may be formed in various areas. In addition, microneedles 120 may be formed in a peripheral area where the openings 111 are not formed.

Referring to FIG. 31, the base film 110 may be provided in a rectangular shape as well as a circular shape. According to an embodiment, the base film 110 may be provided in a rectangular shape. In addition, a plurality of openings 111 may be formed to be spaced apart from each other along the longitudinal direction of the base film 120.

Referring to FIG. 32, the opening 111 may be formed in a slit shape in the longitudinal direction of the base film 110.

Hereinabove, the present invention has been described in detail using preferred embodiments, but the scope of the present invention is not limited to specific embodiments, and should be interpreted according to the appended claims. In addition, those skilled in the art should understand that many modifications and variations are possible without departing from the scope of the present invention.

### [Industrial Applicability]

The microstructure according to the present invention can be used for medical treatment and skin care.

## Claims

1. A multi-material delivery microsystem comprising:
a microstructure in which microneedles are formed on one surface of a base film, and which is made of a first material; and
a material accommodation part which is located in one region of the microstructure and has an accommodation space accommodating a second material different from the first material.

2. The multi-material delivery microsystem of claim 1, wherein the base film has one surface including:
a central area; and
a peripheral area which surrounds the central area and in which the microneedles are formed, and
the material accommodation part is located in the central area of the base film.

3. The multi-material delivery microsystem of claim 2, wherein the accommodation space is located so that the center of the bottom surface of the accommodation space is lower than one surface of the base film.

4. The multi-material delivery microsystem of claim 1, wherein the material accommodation part further includes a ring-shaped partition wall which surrounds the accommodation space and is loacated so that an upper end thereof is lower than tips of the microneedles.

5. The multi-material delivery microsystem of claim 4, wherein the partition wall is provided as a corrugated wall having an upper end located at a first height in an expanded state and a second height lower than the first height in a contracted state.

6. The multi-material delivery microsystem of claim 4, wherein the partition wall includes:
a ring-shaped first partition wall layer; and
a second partition wall layer which is located under the first partition wall layer and has a space formed therein capable of accommodating the first partition wall layer,
wherein the first partition wall layer and the second partition wall layer are switchable between a first state in which they are stacked in multiple stages and a second state in which the first partition wall layer is located inside the second partition wall layer.

7. The multi-material delivery microsystem of claim 4, wherein the partition wall includes:
support parts sequentially positioned to be spaced apart from each other along the circumference of the accommodation space; and
connection parts which are positioned between the support parts and have thicknesses thinner than those of the support parts.

8. The multi-material delivery microsystem of claim 4, wherein the partition wall gradually decreases in diameter and thickness as it goes from the lower end to the upper end.

9. The multi-material delivery microsystem of claim 4, wherein the partition wall gradually increases in diameter and gradually decreases in thickness as it goes from the lower end to the upper end.

10. The multi-material delivery microsystem of claim 4, wherein the material accommodation part further includes a support plate which is located on the bottom of the base film and has the partition wall integrally formed in a central region thereof.

11. The multi-material delivery microsystem of claim 10, wherein the support plate has an inner part in which a storage space for storing the second material is formed, and an opening connecting the storage space and the accommodation space is formed.

12. The multi-material delivery microsystem of claim 10, wherein the support plate has a storage space connected to the accommodation space and formed therein, the bottom surface of the storage space is provided as a film with a thin thickness, and the material accommodation part further includes a pushing part which punches the bottom surface of the storage space and is inserted into the storage space to push the second material stored in the storage space.

13. The multi-material delivery microsystem of claim 10, wherein the support plate has a storage space connected to the accommodation space and formed therein, the bottom surface of the storage space is provided as a film which is convex downward and has a thin thickness, and when the bottom surface of the storage space is pressed from the outside, the bottom surface of the storage space is pressed toward the accommodation space.

14. The multi-material delivery microsystem of claim 1, further comprising:
a membrane in which a plurality of storage spaces partitioned by partition walls are formed in the material accommodation part, and which is positioned in the opening of the base film;
an outflow prevention film blocking an opening formed in the base film between the storage spaces and the membrane; and
a punching rod of which a plurality of ends are located in each of the storage spaces, and which can hit the outflow prevention film,
wherein different materials are stored in each of the storage spaces, and the respective materials are introduced into the membrane through holes formed in the outflow prevention film by hitting of the punching rod and mixed with each other.
